# EUROPEAN PATENT SPECIFICATION

(11) **EP 2 403 522 B1**
(45) Date of publication and mention of the grant of the patent: **16.12.2015**
(21) Application number: 10707756.2
(22) Date of filing: 03.03.2010
(51) Int. Cl.: A61K 38/48, A61P 15/08

(54) **CLOSTRIDIAL TOXIN TO IMPROVE EJACULATE**
CLOSTRIDIUM-TOXIN ZUR VERBESSERUNG DES EJAKULATS
TOXINE CLOSTRIDIENNE POUR AMÉLIORER L'ÉJACULAT

(30) Priority: 06.03.2009 US 158277 P
(43) Date of publication of application: 11.01.2012
(73) Proprietor: ALLERGAN, INC., Irvine, CA 92612 (US)
(72) Inventor: HAAG-MOLKENTELLER, Cornelia, C., Irvine CA 92614 (US); CHEETHAM, Janet, K., Laguna Niguel CA 92607 (US)
(74) Representative: Hoffmann Eitle
(86) International application number: PCT/US2010/025992
(87) International publication number: WO 2010/101968

(56) References cited:
- MIST STUDY GROUP ET AL: "CHANGES IN SEXUAL FUNCTION IN MEN RANDOMIZED INTO A TWO-STAGE PHASE II TRIAL OF 100 AND 300 UNITS BOTULINUM NEUROTOXIN TYPE A (BONT-A) FOR THE MANAGEMENT OF BENIGN PROSTATIC HYPERPLASIA (BPH)" JOURNAL OF UROLOGY, LIPPINCOTT WILLIAMS & WILKINS, BALTIMORE, MD, US LNKD- DOI:10.1016/S0022-5347(09)61491-9, vol. 181, no. 4, 1 April 2009 (2009-04-01) , page 528, XP025979044 ISSN: 0022-5347 [retrieved on 2009-03-12]
- LIM ET AL: "Intraprostatic and Bladder-Neck Injection of Botulinum A Toxin in Treatment of Males with Bladder-Neck Dyssynergia: A Pilot Study" EUROPEAN UROLOGY, ELSEVIER BV, NL LNKD- DOI:10.1016/J.EURURO.2007.10.001, vol. 53, no. 3, 15 October 2007 (2007-10-15), pages 620-627, XP022510087 ISSN: 0302-2838
- CHUANG ET AL: "Botulinum toxin type A improves benign prostatic hyperplasia symptoms in patients with small prostates" UROLOGY, BELLE MEAD, NJ, US LNKD- DOI:10.1016/J.UROLOGY.2005.04.029, vol. 66, no. 4, 1 October 2005 (2005-10-01), pages 775-779, XP005117694 ISSN: 0090-4295

## Description

### Background

Methods for improving ejaculate of a patient in need thereof are presented. More particularly, methods for increasing ejaculate volume/prostatic fluid to increase viability of sperm contained therein, by administration of a neurotoxin to the prostate of the patient are provided.

As is well known, the prostate in human males is located beneath the bladder and ventral to the rectum, encircling a section of the urethra. During ejaculation, prostatic fluid (semen) is secreted by the prostate through small pores of the urethra's walls, which then carry mature sperm out of the body.

The prostate is made up of three lobes, encased by an outer layer, which is flanked on either side by the seminal vesicles. Next to the seminal vesicles run the two vas deferens, tubes that carry sperm from the testicles. These vasa deferrentia pass directly behind the prostate and open into te urethra to propel sperm out into the urethra, in the prostatic section of the urethra. The prostate is divided into the anterior lobe (anterior portion of the gland lying in front of the urethra) median lobe (the cone-shaped portion of the prostate located between the two ejaculatory ducts and the urethra) and the lateral lobes (right and left, separated by the prostatic urethra) that form the main mass of the gland and are continuous posteriorly. The posterior lobe is the postero-medial part of the lateral lobes which is palpated through the rectum during a digital rectal exam.

The prostate plays an important role in the excretion of a prostatic fluid contributing to the overall seminal fluid. The prostatic fluid contributes to about 10-30% of the seminal fluid of an ejaculate. It is mainly composed of proteolytic enzymes, prostate acid phosphatase, and zinc in very high concentrations. Due to its alkaline pH, it is proposed that the prostatic fluid component of the overall seminal fluid protects the sperm against the acidic milieu of the vagina. In addition, the high zinc content may provide additional nutrients contributing to sperm motility. Thus, it can seen that prostatic fluid and its contribution to and as part of the ejaculate play an important part in providing an appropriate milieu conducive to sperm health, including sperm motility and overall condition of the ejaculate, that is, facilitating the overall success of fertilizing an egg. Infertility is typically considered as the inability to conceive after at least one year of unprotected intercourse. Infertility in men can be cause by hormone disorders, illness, reproductive anatomy trauma, obstruction and sexual dysfunction, among other reasons. According to the National Institutes of Health, male infertility is involved in approximately 40% of the 2.6 million infertile married couples in the United States. Common causes for male infertility include impaired sperm production and/or impaired sperm delivery. Investigating a male's fertility typically includes a thorough examination and review of the male's medical and surgical history, which includes a semen analysis and examination of ejaculate, as seminal fluid can affect sperm function and movement, as discussed above. Sperm factors typically considered include concentration (sperm/milliliter), morphology, motility (% sperm movement), testing of thickness and/or color of ejaculate, total motile sperm count and volume of ejaculate. As an example, when a patient's sperm motility count is below 10 million motile sperm per milliliter of ejaculate and/or at least about 50% of the patient's total sperm count is non-motile and/or, for example, if their ejaculate volume is low (e.g. less than about 1 milliliter), the patient may be considered a candidate for infertility treatment.

Botulinum toxin A is known to block the release of acetylcholine at the nerve ending of striated and smooth muscle causing a temporary, reversible muscle relaxation and further effects. For a detailed discussion of botulinum toxin and its prior uses, reference is made to the background sections of U.S. Patents 7,468,189; 7,468,188 and 7,494,654.

Administration and techniques for botulinum toxin administration to the prostate are well known in the literature, including the use of botulinum toxin as an intraprosatic injection to treat benign prostate hyperplasia. A non-exhaustive list of examples of such administration can be found, for example, in U.S. Patent 6,365,164, issued April 2, 2002, and in "The use of botulinum toxin in men with benign prostatic hyperplasia", by Rusnack Susan R and Kaplan Steven A. Rev Urol 2005 Fall;7(4):234-6 and "The application of botulinum toxin in the prostate" by Chuang Yao-Chi and Chancellor Michael B. J Urol 2006; 176(6):2375-82.

What is needed therefore is a simple method by which ejaculate can be improved in a patient in need thereof, whereby prostatic fluid in the patient's ejaculate is increased.

### Summary

In accordance with one aspect of the invention, a method for increasing ejaculate volume and improvement of the ejaculate of a patient in need thereof is provided, where the method comprises a step of local administration of a therapeutically effective amount of a Clostridial neurotoxin to a prostate of the patient, thereby increasing ejaculate volume of the patient. In particular embodiments, this may include local administration of the therapeutic amount of Clostridial neurotoxin to the prostate via transurethrally or transperineally or transrectally routes and in particular instances, for example when administered via transperineally or transrectally, the method can further comprise the step of utilizing ultrasound guidance during administration to the prostate. A particularly useful Clostridial neurotoxin is a botulinum neurotoxin. Exemplary useful botulinum neurotoxins include botulinum neurotoxin types A , B, C, D, E, F and G.

In some embodiments, the botulinum neurotoxin administered is in an amount of from 1 unit to 20,000 units, for example. In a specific embodiment, the botulinum neurotoxin is a type A or type B botulinum neurotoxin, and is administered in an amount of from 1 unit to 3000 units or from 100 to about 10,000 units, respectively. In a preferred embodiment, botulinum neurotoxin type A is utilized.

Local administration of the therapeutic amount of the Clostridial neurotoxin to the prostate of the patient can be accomplished by, but is not limited to, injection of the Clostridial neurotoxin into the prostate. In particular instances, utilization of ultrasound guidance in conjunction with injection of the Clostridial neurotoxin is helpful in order to visualize the prostate, for example, as well as the administration instruments (e.g. needle) during the administration procedure. In a specific example, local administration of the therapeutic amount of the Clostridial neurotoxin to the prostate of the patient is accomplished via injection into at least one lobe of the prostate and/or can also be accomplished via injection into at least the transition zone of the prostate.

Additionally, a method for increasing ejaculate volume to increase viability of sperm contained therein, in a patient in need thereof, the method comprising the step of local administration of a therapeutically effective amount of a botulinum neurotoxin to a prostate of the patient, thereby increasing ejaculate volume and increasing viability of sperm contained therein, is also presented. The botulinum neurotoxin is preferably selected from the group consisting of botulinum neurotoxin types A, B, C, D, E, F and G. More preferably, the neurotoxin is a botulinum neurotoxin type A and is administered via injection to the prostate of the patient. In particular examples, the amount of botulinum neurotoxin type A administered to the prostate is from 1 unit to 2500, more preferably from 100 units to 1000 units of neurotoxin type A, the amount to be administered being determined by the attending medical professional, of course. Varying volumes of compositions containing the botulinum neurotoxin can be administered to the prostate of a patient in need thereof. In a particular example, botulinum neurotoxin is administered to the prostate via introduction of 1 milliliter to 40 milliliters of a botulinum neurotoxin containing composition to the prostate, preferably a botulinum neurotoxin type A. Preferably, from 0.5 milliliter to 10 milliliters of a botulinum neurotoxin containing composition is administered to the prostate in accordance with the present disclosure.

In some instances, multiple sessions of administration of a therapeutically effective amount of a botulinum neurotoxin to the prostate are under taken over a period of time, such as about every two to three months, or about three months after an initial administration to the prostate, or about every three or five or six or nine months, for example. Accordingly and in one example, additional administration of botulinum neurotoxin to the prostate of the patient in need thereof can be performed least 2 months or 3 months after an initial administration of botulinum neurotoxin to the prostate.

In a particular example, a method for increasing ejaculate volume to increase viability of sperm contained therein, in a patient in need thereof is provided, the method comprising the step of local administration of a therapeutically effective amount of a botulinum neurotoxin to a prostate of the patient, where the patient's sperm motility count is below 10 million motile sperm per milliliter of ejaculate or at least about 50% of the patient's total sperm count is non-motile, to thereby increase ejaculate volume and increasing viability of sperm contained therein. In a particular embodiment, botulinum neurotoxin is type A or B, is administered via injection into the prostate in an amount of from 1 to 1500 units or from 50 to 15,000 units, respectively, and in a particular instance, the administration is to at least one lobe of the prostate.

Not wishing to be bound by theory, it can be hypothesized that administration of a botulinum neurotoxin to a prostate of a patient in need thereof can result in relaxation of the periurethral smooth muscles of the prostate and thus could contribute to a more pronounced excretion of prostatic fluid from the glandular positions of the prostate, leading to an increase in the volume of the ejaculate. This ejaculate would then contain more prostatic fluid than an ejaculate from men not having had intraprostatic injections of botulinum neurotoxin, and thus can lead to increased sperm motility and/or quality.

"About" means approximately or nearly and in the context of a numerical value or range set forth herein means ±10% of the numerical value or range recited or claimed.

"Botulinum toxin" means a botulinum neurotoxin as either pure toxin (i.e. about 150 kDa weight molecule) or as a complex (i.e. about 300 to about 900 kDa weight complex comprising a neurotoxin molecule and one or more associated non-toxic molecules), and excludes botulinum toxins which are not neurotoxins such as the cytotoxic botulinum toxins C2 and C3, but can include recombinantly made, hybrid, modified, and chimeric botulinum toxins.

The administration of neurotoxin, such as botulinum neurotoxin, to the prostate is not meant to alter or affect spermatogenesis (i.e. clonal expansion of spermatogonia and subsequent maturation that eventually gives rise to mature sperm), but rather to provide increased prostatic fluid in an ejaculate/increased ejaculate, and thus result in increased/improved characteristics of mature sperm found in the ejaculate. Exemplary improvements can be improved viability and/or motility of sperm contained in the ejaculate as a result of increased volume/increased prostatic fluid in the ejaculate, in accordance with the teachings of the present disclosure.

### Description

The methods disclosed herein are based upon the administration of a neurotoxin, preferably a Clostridial neurotoxin, more preferably a Clostridial botulinum toxin, most preferably a botulinum toxin type A, to the prostate of a patient for which an increase in prostatic fluid and/or ejaculate volume is desired. For example, a patient having a total ejaculate volume of less than about 4 milliliters may be a candidate for the methods herein described for local administration of a botulinum toxin to his prostate in order to increase his ejaculate volume.

In one embodiment, from 1 to 2500 units of a botulinum toxin type A may be locally administered to the prostate utilizing known transurethrally or transperineally or transrectally routes. The use of anesthetic during a particular procedure is left to the attending physical/medical professional to determine. Preferably, the lowest therapeutically effective dosage will be administered to the patient. The lowest therapeutic dosage is that dosage which results in the desired effect on the patient's prostate and ejaculate of the patient to which the neurotoxin is administered. Methods for assessing or quantifying the effect of a toxin on a prostate are well known, as is the measurement of ejaculate volume and sperm count and motility, as determined by those skilled in the art.

In patients where their sperm motility count is below 10 million motile sperm per milliliter of ejaculate or at least about 50% of the patient's total sperm count is non-motile, an increase in prostatic fluid can provide for a better environment (more ejaculate/per sperm) and thus the reduced numbers of sperm can have a better chance and be more in a more motility-conducive/friendly environment and thus have a better chance of surviving and eventually finding and penetrating an egg.

The amount of the Clostridial toxin administered according to a method within the scope of the disclosed invention can vary according to the particular characteristics of the patient being treated, including the volume of ejaculate, size of the prostate and other various patient variables including patient size, weight, age, and responsiveness to therapy. To guide the practitioner and as only a general example, typically, no less than 1 unit and no more than 100 units of a botulinum toxin type A (such as BOTOX®) is administered per injection site (i.e. to each prostate injection site), per patient treatment session. For a botulinum toxin type A such as DYSPORT® no less than 2 units and no more 200 units of the botulinum toxin type A are administered per injection site, per patient treatment session. For a botulinum toxin type B such as MYOBLOC®., no less than 40 units and no more 2000 units of the botulinum toxin type B are administered per injection site, per patient treatment session. As one example, administration can include injection of a total of 200 units of a botulinum toxin type A, in about a 4mL volume of liquid (utilizing an appropriate vehicle, such as sterile water or 0.9% non-preserved sterile saline, for example) equally divided into each lateral prostatic lobe (e.g. 2 mL containing 100 units at two sites per lobe) via a transrectal route of administration.

Although examples of routes of administration (e.g. transurethrally or transperineally or transrectally routes) and the use of ultrasound visualization and dosages are provided, the appropriate route of administration and dosage are generally determined on a case by case basis by the attending physician. Such determinations are routine to one of ordinary skill in the art (see for example, Harrison's Principles of Internal Medicine (1998), edited by Anthony Fauci et al., 14th edition, published by McGraw Hill). As an example, the route and dosage for administration of a Clostridial neurotoxin, preferably a botulinum toxin according to the present disclosure, can be selected based upon criteria such as the solubility characteristics of the neurotoxin chosen as well as the lack of (low) ejaculate volume of the patient. Exemplary methods for administration of botulinum toxin to the prostate are known in the art, examples of which can be found in "The potential and promise of using botulinum toxin in the prostate gland" by Chuang Y-C et al. BJU Int 2006;98(1):28-32 and in "The application of botulinum toxin in the prostate" by Chuang Yao-Chi and Chancellor Michael B. J Urol 2006;176(6):2375-82 and references cited therein.

As stated above, administration of botulinum toxin is thought to result in relaxation of the periurethral smooth muscles. This relaxation could also contribute to a more pronounced excretion of prostatic fluid from the glandular parts of the prostate leading to an increase in the volume of the ejaculate. This ejaculate would then contain more prostatic fluid than an ejaculate from men without intraprostatic injections of botulinum toxin, such as botulinum toxin type A or B, and thus lead to an increased sperm motility and or quality. It is to be understood that typically and as one factor for treatment, having an ejaculate volume of less than 3 milliliters can be indicative of candidacy for intraprostatic administration of a botulinum toxin, as herein disclosed, however the methods and teachings of the present invention are by no means limited to patients having an ejaculate volume of less than 3 milliliters.

### Examples

The following non-limiting examples provide those of ordinary skill in the art with exemplary methods to treat conditions within the scope of the present invention and are not intended to limit the scope of the invention.

### Example 1

A 42 year-old male presents at his doctor's office and reports that after three years of attempting to impregnate his wife, they remain childless. The doctor conducts a full physical and orders analysis of the patient's ejaculate. Tests show that the patient's volume of ejaculate is only about 1.5 milliliters. The doctor decides that local administration of a botulinum neurotoxin to the patient's prostate may result in an increase in ejaculate volume and thus make it more likely for the patient to impregnate his wife.

The doctor proceeds to administer 200 units of botulinum A toxin (BOTOX®) transrectally into the prostate with the appropriate antibiotic coverage. One injection into each of the lateral lobes (100 units per injection site, for a total of 200 units) are made. The patient subsequently presents at the doctor's office and is able to produce ejaculate at a volume of about 4 milliliters and proceeds to successfully impregnate his wife.

### Example 2

A 26 year-old male presents at his doctor's office, reporting that after five years of attempting to have children, he and his wife remain childless. The doctor accordingly orders up an analysis of the patient's ejaculate and the tests reveal that the patient's sperm motility count is below 10 million motile sperm per milliliter of ejaculate and that his ejaculate is of a small volume, here 1 milliiter. The doctor decides to administer 150 units of a botulinum toxin type A (BOTOX®) transperieneully into each of the lateral lobes (150 units of botulinum toxin type A in 2 milliliters of unpreserved sterile normal saline (0.9% sodium chloride)) into each lobe of the prostate. The patient subsequently reports to the doctor's office about 2 months later and the patient is ordered to provide another sample of ejaculate and additional test show that a greater percentage of his sperm appear more motile and the total volume of ejaculate is now 3 milliliters. The doctor, happy with these results, proceeds to administer a second dose of botulinum toxin to the patient, and the patient can subsequently report 3 months later that his wife is pregnant.

### Example 3

A 56 year-old male presents at his doctor's office, and is working on his second marriage. He is reporting that after five years of attempting to have children, he and his younger wife remain childless. The doctor accordingly orders up an analysis of the patient's ejaculate and the tests reveal that the patient's sperm motility count is below 10 million motile sperm per milliliter of ejaculate and that his ejaculate is of a small volume, here 1 milliliter. The doctor decides to administer 150 units of a botulinum toxin type A (BOTOX®) transperienally into each of the lateral lobes (150 units of botulinum toxin type A in 2 milliliters of unpreserved sterile normal saline (0.9% sodium chloride)) into each lobe of the prostate under the appropriate antibiotic coverage. The patient subsequently reports to the doctor's office about 2 months later and the patient is ordered to provide another sample of ejaculate and additional test show that a greater percentage of his sperm appear more motile and the total volume of ejaculate is now 3 milliliters. The doctor, happy with these results, proceeds to administer a second dose of botulinum toxin to the patient, about 6 months later and the patient can subsequently report 3 months later that his wife is pregnant.

As can be seen, patients with a low sperm motility and or decreased sperm quality could potentially benefit from this treatment thus enhancing their ability to actually father a child via intercourse in contrast to having to participate in costly in-vitro fertilization.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability.

Variations of exemplary embodiments will become apparent to those of ordinary skill in the art upon reading the foregoing description. The inventor expects skilled artisans to employ such variations as appropriate, and the inventors intend for the invention to be practiced otherwise than specifically described herein. For example, particular ejection volumes and injection particulars such as number of administration sites and locations of administration to the prostate useful in accordance with the teachings of the present disclosure are considered to be within the scope of the present invention. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein.

## Claims

1. Clostridial neurotoxin for use in a method for increasing ejaculate volume of a patient in need thereof, the method comprising the step of local administration of a therapeutically effective amount of a Clostridial neurotoxin to a prostate of said patient.

2. Clostridial neurotoxin for the use according to claim 1, wherein local administration of said therapeutic amount of Clostridial neurotoxin to the prostate is accomplished transurethrally or transperineally or transrectally, preferably comprising the step of utilizing ultrasound guidance.

3. Clostridial neurotoxin for the use according to claim 1, wherein the Clostridial neurotoxin is a botulinum neurotoxin, preferably selected from the group consisting of botulinum neurotoxin types A, B, C, D, E, F and G.

4. Clostridial neurotoxin for the use according to claim 1, wherein the Clostridial neurotoxin is a botulinum neurotoxin and is administrated in an amount of from 1 unit to 20,000 units.

5. Clostridial neurotoxin for the use according to claim 1, wherein the Clostridial neurotoxin is a botulinum neurotoxin type A or type B, and is administrated in an amount of from 1 unit to 3,000 units or from 100 to 10,000 units respectively.

6. Clostridial neurotoxin for the use according to claim 1, wherein the Clostridial neurotoxin is botulinum neurotoxin type A.

7. Clostridial neurotoxin for the use according to claim 1, wherein the local administration to the of the therapeutic amount of Clostridial neurotoxin to the prostate of patient is accomplished via injection of the Clostridial neurotoxin into the prostate, preferably further comprising the step utilizing ultrasound guidance in conjunction of the Clostridial neurotoxin in order to visualize the prostate.

8. Clostridial neurotoxin for the use in the method of claim 1, wherein local administration of the therapeutic amount of the Clostridial neurotoxin to the prostate of the patient is accomplished via injection into at least one lobe of the prostate or at least the transition zone of the prostate.

9. Clostridial neurotoxin for the use according to claim 3, whereby the method increases the ejaculate volume and the viability of sperm contained therein.

10. Clostridial neurotoxin for use according to claim 9, wherein the botulinum neurotoxin is botulinum neurotoxin type A and is administrated via injection to the prostate of the patient, preferably in an amount of from 1 unit to 2,500 units.

11. Clostridial neurotoxin for use according to claim 9, wherein the botulinum neurotoxin type A is administrated to the prostate via introduction of 1 milliliter to 40 milliliter of a botulinum neurotoxin composition to the prostate.

12. Clostridial neurotoxin for use according to claim 9, further comprising additional administration of a botulinum neurotoxin to the prostate of the patient, at least 2 months after an initial administration of botulinum neurotoxin to the prostate.

13. Clostridial neurotoxin for use according to claim 9, wherein the patient's sperm motility count before treatment is below 10 milion motile sperm per milliliter of ejaculate or at least about 50% of the patient's total sperm count is non-motile.

14. Clostridial neurotoxin for use according to claim 13, wherein the botulinum neurotoxin is type A or B and it is administered via injection to the prostate in an amount of from 1 to 1500 units or from 50 to 15,000 units respectively.

15. Clostridial neurotoxin for use according to claim 14, wherein the administration is to at least one lobe of the prostate.

## Patentansprüche

1. Clostridium-Neurotoxin zur Verwendung in einem Verfahren zur Erhöhung des Ejakulatvolumens eines Patienten, der dies benötigt, wobei das Verfahren den Schritt der lokalen Verabreichung einer therapeutisch wirksamen Menge des Clostridium-Neurotoxins an die Prostata dieses Patienten umfasst.

2. Clostridium-Neurotoxin zur Verwendung gemäss Anspruch 1, wobei die lokale Verabreichung der therapeutischen Menge des Clostridium-Neurotoxins an die Prostata transurethral oder transperineal oder transrektal durchgeführt wird und vorzugsweise einen Schritt umfasst, in dem eine Ultraschallkontrolle verwendet wird.

3. Clostridium-Neurotoxin zur Verwendung gemäss Anspruch 1, wobei das Clostridium-Neurotoxin ein Botulinumneurotoxin ist, das vorzugsweise aus der Gruppe bestehend aus den Botulinumneurotoxin-Typen A, B, C, D, E, F und G ausgewählt ist.

4. Clostridium-Neurotoxin zur Verwendung gemäss Anspruch 1, wobei das Clostridium-Neurotoxin ein Botulinumneurotoxin ist und in einer Menge von 1 bis 20.000 Einheit(en) verabreicht wird.

5. Clostridium-Neurotoxin zur Verwendung gemäss Anspruch 1, wobei das Clostridium-Neurotoxin ein Botulinumneurotoxin vom Typ A oder Typ B ist und in einer Menge von 1 bis 3.000 Einheit(en) bzw. 100 bis 10.000 Einheiten verabreicht wird.

6. Clostridium-Neurotoxin zur Verwendung gemäss Anspruch 1, wobei das Clostridium-Neurotoxin ein Botulinumneurotoxin vom Typ A ist.

7. Clostridium-Neurotoxin zur Verwendung gemäss Anspruch 1, wobei die lokale Verabreichung der therapeutischen Menge des Clostridium-Neurotoxins an die Prostata eines Patienten durch Injektion des Clostridium-Neurotoxins in die Prostata durchgeführt wird und vorzugsweise einen weiteren Schritt umfasst, in dem eine Ultraschallkontrolle in Verbindung mit dem Clostridium-Neurotoxin verwendet wird, um die Prostata sichtbar zu machen.

8. Clostridium-Neurotoxin zur Verwendung in einem Verfahren gemäss Anspruch 1, wobei die lokale Verabreichung der therapeutischen Menge des Clostridium-Neurotoxins an die Prostata eines Patienten durch Injektion in zumindest einen Lappen der Prostata oder zumindest die Übergangszone der Prostata durchgeführt wird.

9. Clostridium-Neurotoxin zur Verwendung gemäss Anspruch 3, wobei das Verfahren das Ejakulatvolumen und die Lebensfähigkeit der darin enthaltenen Spermien erhöht.

10. Clostridium-Neurotoxin zur Verwendung gemäss Anspruch 9, wobei das Botulinumneurotoxin Botulinumneurotoxin vom Typ A ist und durch Injektion in die Prostata des Patienten verabreicht wird.

11. Clostridium-Neurotoxin zur Verwendung gemäss Anspruch 9, wobei das Botulinumneurotoxin vom Typ A durch Einführen von 1 bis 40 ml einer Botulinumneurotoxin-Zusammensetzung in die Prostata verabreicht wird.

12. Clostridium-Neurotoxin zur Verwendung gemäss Anspruch 9, ferner umfassend die zusätzliche Verabreichung eines Botulinumneurotoxins an die Prostata eines Patienten zumindest 2 Monate nach der ersten Verabreichung des Botulinumneurotoxins an die Prostata.

13. Clostridium-Neurotoxin zur Verwendung gemäss Anspruch 9, wobei die Spermienmotilitätszahl des Patienten vor der Behandlung unter 10 Millionen beweglicher Spermien pro Milliliter Ejakulat liegt oder zumindest etwa 50 % der gesamten Spermienzahl des Patienten nicht beweglich ist.

14. Clostridium-Neurotoxin zur Verwendung gemäss Anspruch 13, wobei das Botulinumneurotoxin vom Typ A oder Typ B ist und durch Injektion in einer Menge von 1 bis 1.500 Einheit(en) bzw. 50 bis 15.000 Einheiten an die Prostata verabreicht wird.

15. Clostridium-Neurotoxin zur Verwendung gemäss Anspruch 14, wobei die Verabreichung an zumindest einen Lappen der Prostata erfolgt.

## Revendications

1. Neurotoxine clostridienne à utiliser dans un procédé pour augmenter le volume de l'éjaculat d'un patient en ayant besoin, le procédé comprenant l'étape d'administration locale d'une quantité thérapeutiquement efficace d'une neurotoxine clostridienne à la prostate dudit patient.

2. Neurotoxine clostridienne à utiliser selon la revendication 1, dans laquelle l'administration locale de ladite quantité thérapeutique de neurotoxine clostridienne à la prostate est réalisée par voie transurétrale ou transpérinéale ou transrectale, comprenant de préférence l'étape d'utilisation d'un guidage par échographie.

3. Neurotoxine clostridienne à utiliser selon la revendication 1, dans laquelle la neurotoxine clostridienne est une neurotoxine botulique, choisie de préférence dans le groupe constitué par les types de neurotoxine botulique A, B, C, D, E, F et G.

4. Neurotoxine clostridienne à utiliser selon la revendication 1, dans laquelle la neurotoxine clostridienne est une neurotoxine botulique et est administrée en une quantité de 1 unité à 20 000 unités.

5. Neurotoxine clostridienne à utiliser selon la revendication 1, dans laquelle la neurotoxine clostridienne est une neurotoxine botulique de type A ou B, et est administrée en une quantité d'environ 1 unité à 3 000 unités ou de 100 à 10 000 unités, respectivement.

6. Neurotoxine clostridienne à utiliser selon la revendication 1, dans laquelle la neurotoxine clostridienne est une neurotoxine botulique de type A.

7. Neurotoxine clostridienne à utiliser selon la revendication 1, dans laquelle l'administration locale de la quantité thérapeutique de la neurotoxine clostridienne à la prostate du patient est réalisée par l'intermédiaire d'une injection de la neurotoxine clostridienne dans la prostate, comprenant en outre de préférence, l'étape d'utilisation d'un guidage par échographie conjointement à la neurotoxine clostridienne afin de visualiser la prostate.

8. Neurotoxine clostridienne à utiliser dans le procédé selon la revendication 1, dans laquelle l'administration locale de la quantité thérapeutique de la neurotoxine clostridienne à la prostate du patient est réalisée par l'intermédiaire d'une injection dans au moins un lobe de la prostate ou au moins la zone de transition de la prostate.

9. Neurotoxine clostridienne à utiliser selon la revendication 3, par quoi le procédé augmente le volume de l'éjaculat et la viabilité des spermatozoïdes contenus dans celui-ci.

10. Neurotoxine clostridienne à utiliser selon la revendication 9, dans laquelle la neurotoxine botulique est une neurotoxine botulique de type A et est administrée par l'intermédiaire d'une injection à la prostate du patient, de préférence en une quantité de 1 unité à 2 500 unités.

11. Neurotoxine clostridienne à utiliser selon la revendication 9, dans laquelle la neurotoxine botulique de type A est administrée à la prostate par l'intermédiaire de l'introduction de 1 millilitre à 40 millilitres d'une composition de neurotoxine botulique à la prostate.

12. Neurotoxine clostridienne à utiliser selon la revendication 9, comprenant en outre l'administration supplémentaire d'une neurotoxine botulique à la prostate du patient, au moins 2 mois après une administration initiale de neurotoxine botulique à la prostate.

13. Neurotoxine clostridienne à utiliser selon la revendication 9, dans laquelle le comptage de la motilité wdes spermatozoïdes du patient avant le traitement est inférieur à 10 millions de spermatozoïdes mobiles par millilitre d'éjaculat ou au moins environ 50%% du nombre total de spermatozoïdes du patient est non mobile.

14. Neurotoxine clostridienne à utiliser selon la revendication 13, dans laquelle la neurotoxine botulique est de type A ou B, et elle est administrée par l'intermédiaire d'une injection à la prostate en une quantité de 1 à 1 500 unités ou de 50 à 15 000 unités, respectivement.

15. Neurotoxine clostridienne à utiliser selon la revendication 14, dans laquelle l'administration s'effectue à au moins un lobe de la prostate.
